# EUROPEAN PATENT APPLICATION

(11) **EP 1 776 991 A1**
(43) Date of publication of application: **25.04.2007**
(21) Application number: 04771832.5
(22) Date of filing: 12.08.2004
(51) Int. Cl.: B01D 35/01, A61J 1/00, A61K 35/14, A61M 1/02

(54) **FILTER DEVICE AND FILTERING METHOD**

(71) Applicant: JMS Co., Ltd., Hiroshima-shi, Hiroshima 730-8652 (JP); JMS Singapore Pte Ltd., Singapore 569620 (SG)
(72) Inventor: YANG, Chek Lon c/o JMS SINGAPORE PTE LTD, Singapore 569620 (SG)
(74) Representative: Schwarzensteiner, Marie-Luise
(86) International application number: PCT/JP2004/011871
(87) International publication number: WO 2006/016421

(57) **Abstract**

Filtration equipment is used that includes: a filter 2 for filtering a liquid filling a first container 1; a second container 3 for collecting the liquid that has been filtered through the filter 2; a third container 7; a first line 4 for connecting the first container 1 and the filter 2 to each other; a second line 5 for connecting the filter 2 and the second container 3 to each other; and a third line 6 that branches off from the second line 5 and is connected to the third container 7. The third container 7 is placed above the second container 3, with a part of the third container 7 connected to the third line 6 facing downward. The second container 3 is placed below the first container 1 and is formed so as to be changed in shape by application of external force and to be restored by removal of the external force.

## Description

### TECHNICAL FIELD

The present invention relates to filtration equipment and a filtration method, particularly to filtration equipment and a filtration method that are applicable to whole blood, various preparations derived from blood components contained in whole blood, and biological fluids.

### BACKGROUND ART

Recently, whole blood collected from donors rarely is used for blood transfusion without being treated, while blood component transfusion is administered in many cases. Hence, generally, whole blood collected from donors is divided into various components such as a blood cell component, a blood platelet component, and a blood plasma component, which then are used for various blood preparations.

It has been known that when leukocytes are contained in such blood preparations, a patient subjected to a blood transfusion treatment suffers various side effects. Recently, whole blood collected from donors, therefore, is filtered using a leukocyte removal filter (see, for instance, U.S. Patent No. 6,086,770).

Furthermore, when air is contained in filtered blood, blood components are agglutinated for example. Hence, in the filtration process for removing leukocytes, undesired air is removed from filtered blood by various methods.

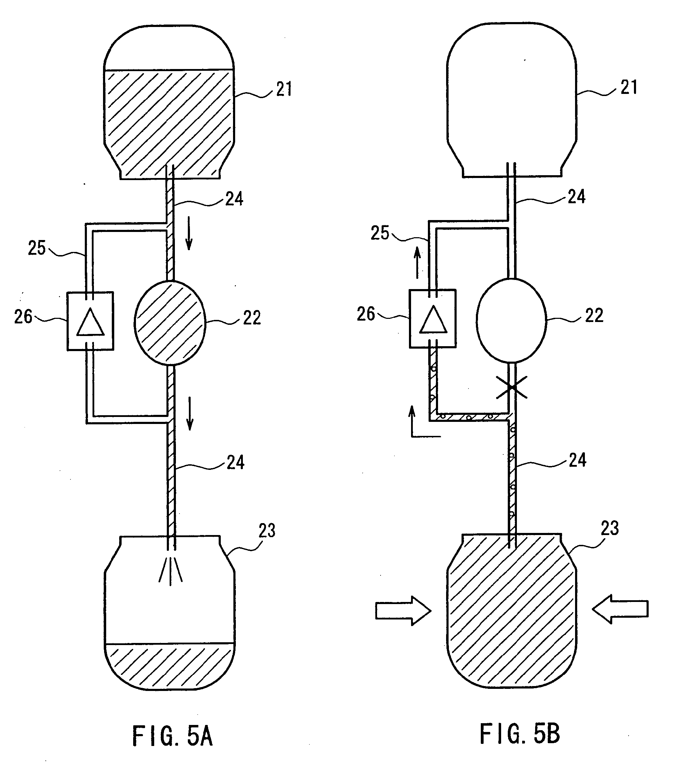

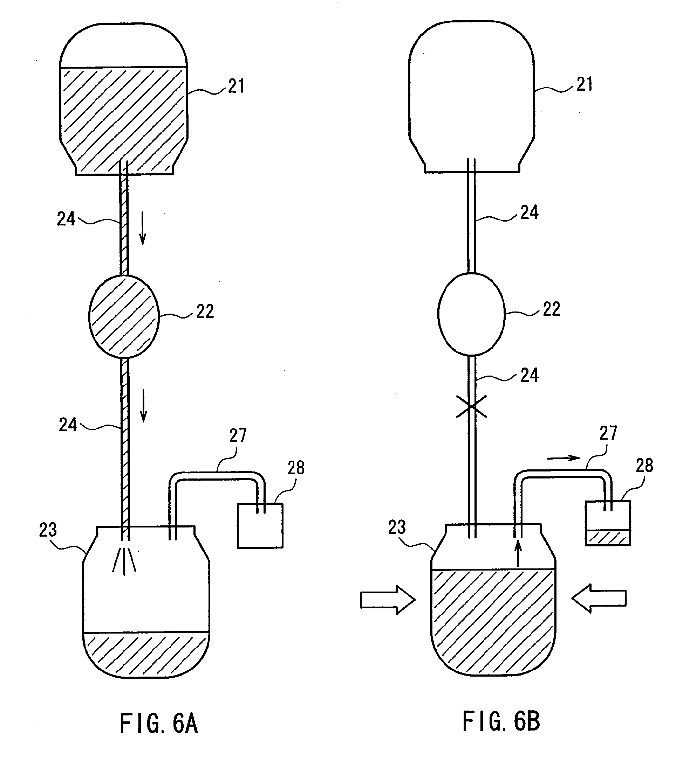

A conventional filtration process for removing leukocytes (a leukocyte removal process) is described with reference to the drawings. FIGs. 5A to 7 are schematic views showing examples of the conventional leukocyte removal process. FIGs. 5A and 5B as well as FIGs. 6A and 6B show a series of processes, respectively. The following descriptions are made with reference to the respective drawings.

In the example shown in FIGs. 5A and 5B, the leukocyte removal process is carried out by sending whole blood filling a first blood bag 21 to a leukocyte removal filter 22 as shown in FIG. 5A. Filtered blood is collected in a second blood bag 23.

In FIGs. 5A and 5B, numeral 24 indicates a blood line for connecting the first blood bag 21 and the second blood bag 23 to each other. The leukocyte removal filter 22 is provided on the blood line 24. The first blood bag 21 is placed above the second blood bag 23. Accordingly, the whole blood falls by gravity to be sent to the leukocyte removal filter 22.

In the example shown in FIGs. 5A and 5B, a bypass line 25 is provided in parallel with the blood line 24 to remove air. The bypass line 25 is provided with a check valve 26 that allows air alone to pass therethrough in one direction (upwards in FIGs. 5A and 5B).

In the example shown in FIGs. 5A and 5B, air is removed as follows. First, as shown in FIG. 5B, the blood line 24 is clamped so that the filtered blood that has been collected in the second blood bag 23 does not flow into the blood removal filter 22. Thereafter, pressing force is applied to the second blood bag 23 to send the filtered blood to the bypass line 25, and thereby air that is contained in the blood is removed by the check valve 26.

Similarly in the example shown in FIGs. 6A and 6B, the leukocyte removal process is carried out using a leukocyte removal filter 22 as shown in FIG. 6A. In the example shown in FIGs. 6A and 6B, however, air is removed using an air removal container 28. The air removal container 28 is connected to a second blood bag 23 through an air vent line 27. Specifically, pressing force is applied to the second blood bag 23, with the blood line 24 being clamped, and thereby undesired air is sent to the air removal container 28.

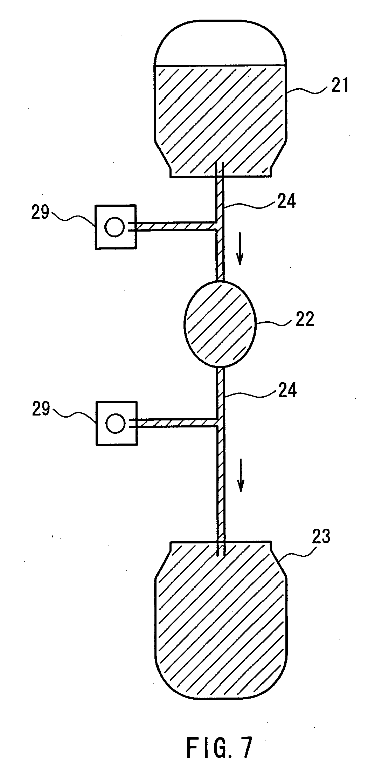

Similarly in the example shown in FIG. 7, the leukocyte removal process is carried out using a leukocyte removal filter 22. In the example shown in FIG. 7, however, air removal filters 29 are provided for the blood line 24 between a first blood bag 21 and the leukocyte removal filter 22 as well as between the leukocyte removal filter 22 and a second blood bag 23.

The air removal filters 29 are vent filters that prevent liquid from passing therethrough so that gas alone can pass therethrough. Accordingly, in the example shown in FIG. 7, by sending the blood filling the first blood bag 21 to the second blood bag 23, air contained in the blood is discharged from outlets (not shown) of the respective air removal filters 29. In other words, while the leukocyte removal process is carried out, undesired air also can be removed.

When the air removal filters 29 are not in use, caps are attached to the outlets (not shown) of the respective air removal filters 29. Because of the structure of the air removal filters, dust and the like are liable to enter the air removal filters from their outlets. Thus, the caps are attached in order to prevent the entry of the dust and the like.

As described above, when any one of the leukocyte removal processes show in FIGs. 5A to 7 is carried out, blood from which not only leukocytes but also undesired air has been removed can be obtained.

In the example shown in FIGs. 5A and 5B, however, a rather strong pressing force needs to be applied to the second blood bag 23 in order to remove air. This may cause a portion of the blood to flow into the check valve 26 together with air frequently. Moreover, the blood that has flowed into the check valve 26 cannot flow backwards through the check valve 26. Accordingly, there is a problem of loss of blood. Furthermore, in the example shown in FIGs. 5A and 5B, when the check valve 26 does not function, there is a risk that filtered blood might be contaminated with blood that has not yet been filtered.

Similarly in the example shown in FIGs. 6A and 6B, it is difficult to remove air alone and a portion of blood is sent to the air removal container 28 together with air, which causes a problem of loss of the blood.

Furthermore, in the example shown in FIGs. 6A and 6B, by applying pressing force to the air removal container 28 or by inverting the air removal container 28, it is possible to send blood that has been sent to the air removal container 28, back to the second blood bag 23. In this case, however, air also is sent back together with the blood, thereby arising the necessity of removing air again. Thus, it is difficult to solve the above-mentioned problem after all.

Furthermore, in the example shown in FIG. 7, there is a problem in that the user has to perform laborious procedures of detaching/attaching the caps from/to the outlets of the air removal filters in order to prevent the entry of dust and the like from the outlets. Also, there is a problem in that, even though the caps are attached to the outlets of the air removal filters when the air removal filters are not in use, dust and the like may enter from the outlets when detaching the caps for the leukocyte removal process. In addition, there is a problem in that it is necessary to provide two or more air removal filters 29 for sufficient removal of air, which results in high cost.

### DISCLOSURE OF THE INVENTION

The present invention is intended to solve the above-mentioned problem and to provide filtration equipment and a filtration method that allow gas to be removed from a liquid easily while suppressing contamination or loss of the liquid.

In order to achieve the above-mentioned objective, filtration equipment of the present invention is used for filtering a liquid filling a first container and includes: a filter for filtering the liquid filling the first container; a second container for collecting the liquid that has been filtered through the filter; a third container; a first line for connecting the first container and the filter to each other; a second line for connecting the filter and the second container to each other; and a third line that branches off from the second line and is connected to the third container. The filtration equipment is characterized in that the third container is placed above the second container, with a part of the third container connected to the third line facing downward, and the second container is placed below the first container and is formed so as to be changed in shape by application of external force and to be restored by removal of the external force.

Furthermore, in order to achieve the above-mentioned objective, the filtration method of the present invention uses filtration equipment including: a filter for filtering a liquid filling a first container; a second container for collecting the liquid that has been filtered through the filter; a third container; a first line for connecting the first container and the filter to each other; a second line for connecting the filter and the second container to each other; and a third line that branches off from the second line and is connected to the third container. In the filtration equipment, the third container is placed above the second container, with a part of the third container connected to the third line facing downward, and the second container is placed below the first container and is formed so as to be changed in shape by application of external force and to be restored by removal of the external force. The filtration method is characterized in including at least: (a) sending the liquid filling the first container to the second container through the filter; (b) closing the second line between the filter and a branching point of the third line to send out the liquid that has been filtered to be collected in the second container, to the third container; (c) separating, in the third container, gas that is contained in the liquid that has been filtered, from the liquid sent out to the third container; and (d) sending the liquid that has been filtered and has been sent out to the third container, back to the second container.

### BRIEF DESCRIPTION OF DRAWINGS

FIG. 1 is a diagram showing the configuration of an example of the filtration equipment according to the present invention.
FIG. 2 is a diagram showing a step in an example of the filtration method according to the present invention.
FIG. 3 is a diagram showing a step following the step shown in FIG. 2.
FIG. 4 is a diagram showing a step following the step shown in FIG. 3.
FIGs. 5A and 5B are schematic views showing an example of conventional leukocyte removal process and show a series of processes.
FIGs. 6A and 6B are schematic views showing an example of conventional leukocyte removal process and show a series of processes.
FIG. 7 is a schematic view showing an example of conventional leukocyte removal process.

### BEST MODE FOR CARRYING OUT THE INVENTION

In the above-mentioned filtration equipment according to the present invention, it is preferable that the filtration equipment further includes a first shutoff device and a second shutoff device that close the second line, and the first shutoff device is provided for the second line between the filter and a branching point of the third line while the second shutoff device is provided for the second line between the branching point and the second container. This configuration facilitates the removal of air in the filtration equipment of the present invention.

In the above-mentioned filtration method according to the present invention, it is preferable that in process (b), pressing force is applied to the second container from outside to change it in shape and thereby the liquid that has been filtered is sent out to the third container, and in process (d), the liquid that has been filtered is sent back to the second container by a release of the pressing force. This allows the filtration method of the present invention to be carried out easily.

Furthermore, in the above-mentioned filtration equipment and filtration method of the present invention, it is preferable that the liquid to be subjected to filtration is whole blood collected from a human body, a preparation derived from a blood component contained in the whole blood, or a biological fluid. Furthermore, it is preferable that the filter is a leukocyte removal filter for removing leukocytes that are contained in the whole blood, the preparation, or the biological fluid.

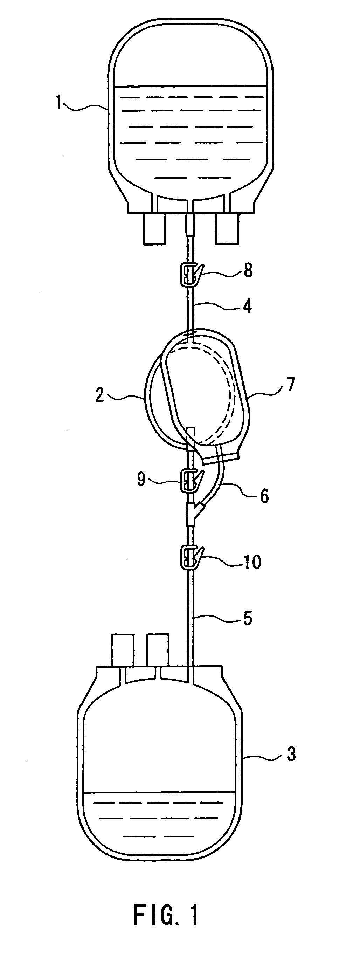

Hereinafter, the filtration equipment and filtration method of the present invention are described with reference to FIGs. 1 to 4. First, an example of the filtration equipment according to the present invention is described with reference to FIG. 1. In the filtration equipment shown in FIG. 1, whole blood collected from a human body is subjected to a filtration process. The filtration equipment carries out a process of removing leukocytes that are contained in whole blood as a filtration process. In the present example, a first container 1 is filled with the whole blood.

While the following description is directed to the examples where whole blood is used as a liquid to be subjected to filtration, it is to be noted that preparations derived from blood components (blood component preparations, plasma preparations, etc.) and biological fluids also can be used as a liquid to be subjected to filtration in the examples shown in FIGS. 1 to 4.

As shown in FIG. 1, the filtration equipment includes a filter 2, a second container 3 where liquid that has been filtered is collected, and a third container 7. In the present example, the filter 2 is a leukocyte removal filter that removes leukocytes that are contained in whole blood, a preparation derived from a blood component, or a biological fluid.

The filtration equipment also is provided with a first line 4, a second line 5, and a third line 6. The first line 4 connects the first container 1 and the filter 2 to each other. The second line 5 connects the filter 2 and the second container 3 to each other. The third line 6 is a line that branches off from the second line 5 and is connected to the third container 7. In the present example, the first line 4, the second line 5, and the third line 6 each are formed of a flexible tube.

As shown in FIG. 1, the second container 3 is placed below the first container 1. Accordingly, the whole blood filling the first container 1 falls due to gravity to be sent to the filter 2 and then to the second container 3. In the present example, the filter 2 is placed above the second container.

Furthermore, as shown in FIG. 1, the third container 7 is placed above the second container 3, with a part of the third container 7 connected to the third line facing downward. In the present example, the third container 7 is attached to the first line 4 to be located at the same level as that of the filter 2.

In the present example, the filtration equipment includes a first shutoff device 9, a second shutoff device 10, and a third shutoff device 8. The third shutoff device 8 is provided for the first line 4. The first shutoff device 9 is provided for the second line 5 between the branching point of the third line 6 and the filter 2. Furthermore, the second shutoff device 10 is provided for the second line 5 between the branching point and the second container 3.

The first shutoff device 9, the second shutoff device 10, and the third shutoff device 8 are clamps for medical use. They press the tubes forming the lines to close them. These shutoff devices are not limited as long as they can close the lines. Beside the clamps, they may be, for instance, valves that can be opened and closed arbitrarily.

In the present example, the second container 3 is a medical bag formed of a resin film. Hence, the second container 3 can be changed in shape by application of external force such as force that is applied by a human hand and also can be restored by removal of the external force.

Furthermore, in the present example, the first container 1 and the third container 7 also are medical bags formed of a resin film. In the present invention, however, the first container 1 and the third container 7 may be, for example, medical bottles formed of a material that is not changed easily in form by application of force that is exerted by a human hand.

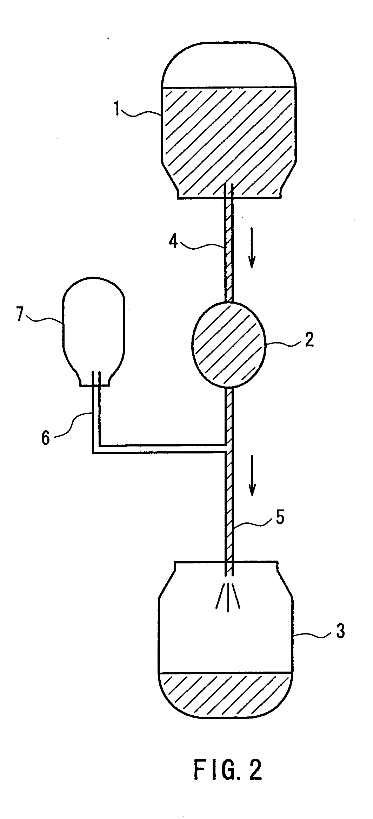

An example of the filtration method according to the present invention is described with reference to FIGs. 2 to 4. FIGs. 2 to 4 show successive steps in the example of the filtration method according to the present invention. The filtration method shown in FIGs. 2 to 4 is carried out using the filtration equipment shown in FIG. 1. In FIGs. 2 to 4, the filtration equipment is shown simplistically. The following description is made with suitable reference to FIG. 1.

First, as shown in FIG. 2, the third shutoff device 8, the first shutoff device 9, and the second shutoff device 10 (see FIG. 1) all are opened to allow whole blood filling the first container 1 to be sent to the filter 2 and then to the second container 3. Consequently, the whole blood filling the first container 1 is collected in the second container 3 after leukocytes that are contained in the whole blood are removed by the filter 2.

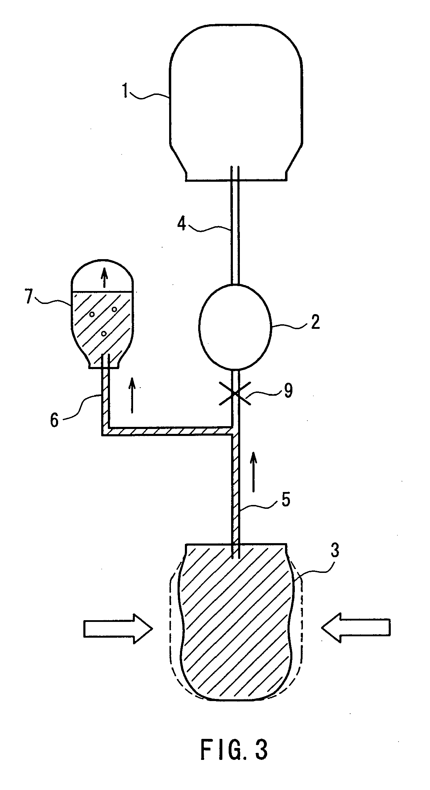

Next, as shown in FIG. 3, the first shutoff device 9 closes the second line 5 (see FIG. 1) between the filter 2 and the branching point. In this state, the second container 3 is pressed to be changed in form. This allows the filtered whole blood that has been collected in the second container 3 to be sent out to the third container 7 through the third line 6.

Further, when the state shown in FIG. 3 where the second container 3 is being pressed is maintained, air contained in the filtered whole blood and air accumulated in the second container 3 gradually gather upwards inside the third container 7. As a result, air is separated from the filtered whole blood.

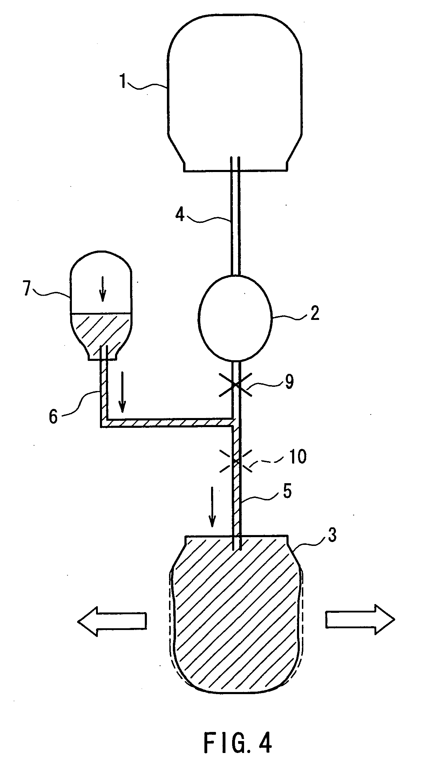

Subsequently, as shown in FIG. 4, the force of pressing the second container 3 is released and thereby the external force exerted on the second container 3 is removed. This allows the filtered whole blood that has been sent out to the third container 7 to start flowing back to the second container 3 by gravity since the third container 7 is placed above the second container 3. Thus, the shape of the second container 3 is restored gradually.

Next, when the interface between the filtered whole blood and air reaches a level between the branching point and the second container 3, the second shutoff device 10 (see FIG. 1) closes the second line 5 between the branching point and the second container 3. When the second line 5 is closed with the second shutoff device 10, considerations are given to preventing the air from flowing back into the second container 3 and preventing the filtered whole blood from remaining in the second line 5 as much as possible. Thereafter, the second container 3 is detached from the second line 5.

As described above, according to the filtration equipment and filtration method of the present examples, air can be removed easily from the filtered whole blood while suppressing the loss of the whole blood. Moreover, since the filtration equipment and filtration method of the present examples do not use a vent filter, the contamination of the whole blood also is suppressed.

When the filtration is to be continued using another second container 3, the second line 5 may be closed with the second shutoff device 10 (see FIG. 1) with consideration given only to preventing the air from flowing back into the second container 3. In this case, since the filtered whole blood that has remained in the second line 5 is collected in another second container 3, the filtered whole blood is not wasted. Furthermore, when the filtered blood is to be evaluated, the second shutoff device 10 (see FIG. 1) may be closed, with a necessary amount of blood remaining in the second line 5 as an evaluation sample.

The filtration equipment and filtration method of the present invention can be used not only for a process of removing leukocytes but also for, for instance, any processes that require removal of air contained in a liquid. Examples of such processes include various processes carried out during the manufacture of blood preparations and biological fluids. Examples of the various processes include pathogen inactivation, purification, and recombination using a biological preparation or a drug. Moreover, according to the filtration equipment and filtration method of the present invention, not only whole blood, preparations derived from blood components, and biological fluids but also other substances such as medical fluids, for example, can be used as a liquid to be subjected to filtration.

### INDUSTRIAL APPLICABILITY

As described above, the filtration equipment and filtration method of the present invention allow gas that is contained in a liquid to be removed therefrom easily while suppressing contamination or loss of the liquid. For example, when the filtration equipment and filtration method of the present invention are used for, for instance, a process of removing leukocytes from a liquid such as whole blood, a preparation derived from a blood component, or a biological fluid, they allow air that is contained therein to be removed while suppressing contamination or loss of the filtered liquid. Thus, the present invention can contribute to the solution to lack in blood preparations used for blood transfusion, for example.

## Claims

1. Filtration equipment for filtering a liquid filling a first container, the filtration equipment comprising:
a filter for filtering the liquid filling the first container;
a second container for collecting the liquid that has been filtered through the filter;
a third container;
a first line for connecting the first container and the filter to each other;
a second line for connecting the filter and the second container to each other; and
a third line that branches off from the second line and is connected to the third container,
wherein the third container is placed above the second container, with a part of the third container connected to the third line facing downward, and
the second container is placed below the first container and is formed so as to be changed in shape by application of external force and to be restored by removal of the external force.

2. The filtration equipment according to claim 1, further comprising a first shutoff device and a second shutoff device that close the second line,
wherein the first shutoff device is provided for the second line between the filter and a branching point of the third line while the second shutoff device is provided for the second line between the branching point and the second container.

3. The filtration equipment according to claim 1 or 2, wherein the liquid filling the first container is whole blood collected from a human body, a preparation derived from a blood component contained in the whole blood, or a biological fluid, and the filter is a leukocyte removal filter that removes leukocytes that are contained in the whole blood, the preparation, or the biological fluid.

4. A filtration method using filtration equipment comprising: a filter for filtering a liquid filling a first container; a second container for collecting the liquid that has been filtered through the filter; a third container; a first line for connecting the first container and the filter to each other; a second line for connecting the filter and the second container to each other; and a third line that branches off from the second line and is connected to the third container, the third container being placed above the second container, with a part of the third container connected to the third line facing downward, and the second container being placed below the first container and being formed so as to be changed in shape by application of external force and to be restored by removal of the external force, the filtration method comprising:
(a) sending the liquid filling the first container to the second container through the filter;
(b) closing the second line between the filter and a branching point of the third line to send out the liquid that has been filtered to be collected in the second container, to the third container;
(c) separating, in the third container, gas that is contained in the liquid that has been filtered, from the liquid sent out to the third container; and
(d) sending the liquid that has been filtered and has been sent out to the third container, back to the second container.

5. The filtration method according to claim 4, wherein in process (b), pressing force is applied to the second container from outside to change it in shape and thereby the liquid that has been filtered is sent out to the third container, and
in process (d), the liquid that has been filtered is sent back to the second container by a release of the pressing force.
